# EUROPEAN PATENT APPLICATION

(11) **EP 4 498 085 A1**
(43) Date of publication of application: **29.01.2025**
(21) Application number: 23775311.6
(22) Date of filing: 22.03.2023
(51) Int. Cl.: G01N 33/50

(54) **COMPOSITION FOR PREVENTION OR TREATMENT OF CANCER COMPRISING DDX54 INHIBITOR AND CANCER IMMUNOTHERAPY AGENT**

(30) Priority: 23.03.2022 KR 20220036242
(71) Applicant: Korea Advanced Institute of Science and Technology, Daejeon 34141 (KR)
(72) Inventor: CHO, Kwang Hyun, Daejeon 34141 (KR); LEE, Jung Eun, Daejeon 34141 (KR); GONG, Jeong Ryeol, Daejeon 34141 (KR)
(74) Representative: Potter Clarkson
(86) International application number: PCT/KR2023/003819
(87) International publication number: WO 2023/182815

(57) **Abstract**

The present invention relates to a pharmaceutical composition for the prevention or treatment of cancer, comprising a DEAD-box helicase 54 (DDX54) inhibitor and a cancer immunotherapy agent, and to a combination therapy of cancer immunotherapy. The DDX54 inhibitor according to the present invention can enhance the efficacy of cancer immunotherapy when used in combination with a cancer immunotherapy agent, and the composition containing the DDX54 inhibitor and a cancer immunotherapy agent inhibits proliferation of cancer, which is a key characteristic thereof, and simultaneously inhibits a signal transduction pathway known as an immune evasion mechanism, and thus may be provided for the prevention or treatment of cancer.

## Description

### [Technical Field]

The present invention relates to a pharmaceutical composition for the prevention or treatment cancer, including a DEAD-box Helicase 54 (DDX54) inhibitor and a cancer immunotherapy agent, and a combination therapy of cancer immunotherapy.

### [Background Art]

Cancer immunotherapy is a cancer treatment strategy that induces immune cells to selectively attack cancer cells by activating the immune system in the human body. Unlike conventional treatments that target self-characteristics of cancer cells, the cancer immunotherapy shows sustainable efficacy and the possibility of complete cure in various types of cancer by enhancing the functions of immune cells and controlling a tumor microenvironment and thus has recently been attracting attention as a new paradigm in cancer treatment. Among these, an immune checkpoint inhibitor targeting PD-1/PD-L1 is known to exhibit a high effect relative to therapeutic toxicity by inhibiting PD-1/PD-L1, which is expressed in most tumors and minimally expressed in non-lymphoid tissues under normal physiological conditions.

However, although the cancer immunotherapy is attracting attention as a new cancer treatment strategy, there is a limitation in that the response rate is low at only an average of 20% in a patient group showing therapeutic effects of a cancer immunotherapy agent. Accordingly, to overcome the limitation, biomarkers capable of predicting the responsiveness to cancer immunotherapy have been actively developed. High tumor mutational burden (TMB-H; TMB > 10 per megabase) was approved by the FDA in 2020 as a biomarker for predicting responsiveness to an immune checkpoint inhibitor that may be applied to solid cancer regardless of tissues. The high tumor mutational burden (TMB) means that many somatic mutations occur to generate neoantigens that are different from normal antigens, which are presented on the cell surface and may be eliminated by immune cells such as T cells.

Therefore, when the tumor mutation burden is high, a high therapeutic effect of immune checkpoint inhibitors may be expected due to an increase in tumor-infiltrating lymphocytes. However, prediction of complete responsiveness has not yet been realized, and there is the need for development of combination therapy with immune checkpoint inhibitors capable of improving therapeutic effects in addition to the development of biomarkers.

### [Disclosure]

### [Technical Problem]

Under the above-mentioned technical background, the present inventors identified a key regulator DDX54 (DEAD-box Helicase 54) in a patient group with low responsiveness to cancer immunotherapy by applying a tumor mutational burden (TIL), which was approved by the FDA as a biomarker for predicting the responsiveness to cancer immunotherapy, verified that the DDX54 expression was inhibited to exhibit a synergistic effect in enhancing a cancer treatment effect of the cancer immunotherapy agent, and then completed the present invention.

An object of the present invention is to provide a pharmaceutical composition for the prevention or treatment of cancer using inhibition of DEAD-box Helicase 54 (DDX54) expression.

Another object of the present invention is to provide a composition for enhancing the efficacy of cancer immunotherapy using inhibition of DEAD-box Helicase 54 (DDX54) expression.

Yet another object of the present invention is to provide a method for enhancing the efficacy of cancer immunotherapy or a method for treating cancer using inhibition of DEAD-box Helicase 54 (DDX54) expression.

Still another object of the present invention is to provide a combination agent for enhancing the activity of an immune checkpoint inhibitor using inhibition of DEAD-box Helicase 54 (DDX54) expression.

### [Technical Solution]

In order to achieve the objects, an aspect of the present invention provides a pharmaceutical composition for the prevention or treatment of cancer including a DEAD-box Helicase 54 (DDX54) inhibitor and a cancer immunotherapy agent.

The DDX54 inhibitor may be at least one selected from the group consisting of antisense nucleotide, siRNA, shRNA, ribozyme, aptamer, micro RNA mimic, nuclease, ZFNs, TALENs, gRNA, sgRNA, peptides, peptide mimics, substrate analogs, antibodies or fragments thereof, and vectors expressing the same, which target the DDX54 protein or a gene encoding the same.

The cancer may have resistance to the cancer immunotherapy agent.

The DDX54 inhibitor may reduce resistance of cancer cells to the cancer immunotherapy agent.

The cancer immunotherapy agent may be an immune checkpoint inhibitor, an immune cell therapy, or an immune virus therapy.

The immune checkpoint inhibitor may be at least one selected from the group consisting of a PD-1 inhibitor, a PDL-1 inhibitor, a PD-L2 inhibitor, a CTLA-4 inhibitor, a LAG-3 inhibitor, a TIM-3 inhibitor, a CEACAM1 inhibitor, a CD27 inhibitor, a CD28 inhibitor, a CD70 inhibitor, a CD80 inhibitor, a CD86 inhibitor, a CD137 inhibitor, a CD276 inhibitor, a KIRs inhibitor, a LAG3 inhibitor, a TNFRSF4 inhibitor, a GITR inhibitor, a GITRL inhibitor, a 4-1BBL inhibitor, a A2AR inhibitor, a VTCN1 inhibitor, a BTLA inhibitor, a IDO inhibitor, a TIM-3 inhibitor, a VISTA inhibitor, a KLRA inhibitor, and combinations thereof.

The composition may inhibit the activity of at least one signal transduction pathway selected from the group consisting of JAK-STAT3, MYC, STEMNESS, EMT, and WNT.

The composition may increase tumor-infiltrating lymphocytes (TILs).

The cancer may be solid cancer.

The cancer may be at least one selected from the group consisting of breast cancer, head and neck cancer, uterine cancer, brain cancer, skin cancer, kidney cancer, lung cancer, colorectal cancer, prostate cancer, liver cancer, bladder cancer, kidney cancer, pancreatic cancer, thyroid cancer, esophageal cancer, eye cancer, stomach cancer, ovarian cancer, cervical cancer, gallbladder cancer, bile duct cancer, and osteosarcoma.

Another aspect of the present invention provides a composition for enhancing the efficacy of cancer immunotherapy including a DEAD-box Helicase 54 (DDX54) inhibitor.

The composition may reduce the resistance of cancer to the cancer immunotherapy agent and enhance anti-tumor immune responses.

The cancer immunotherapy agent may be an immune checkpoint inhibitor.

The immune checkpoint inhibitor may be at least one selected from the group consisting of a PD-1 inhibitor, a PDL-1 inhibitor, a PD-L2 inhibitor, a CTLA-4 inhibitor, a LAG-3 inhibitor, a TIM-3 inhibitor, a CEACAM1 inhibitor, a CD27 inhibitor, a CD28 inhibitor, a CD70 inhibitor, a CD80 inhibitor, a CD86 inhibitor, a CD137 inhibitor, a CD276 inhibitor, a KIRs inhibitor, a LAG3 inhibitor, a TNFRSF4 inhibitor, a GITR inhibitor, a GITRL inhibitor, a 4-1BBL inhibitor, a A2AR inhibitor, a VTCN1 inhibitor, a BTLA inhibitor, a IDO inhibitor, a TIM-3 inhibitor, a VISTA inhibitor, a KLRA inhibitor, and combinations thereof.

Yet another aspect of the present invention provides a method for enhancing the efficacy of cancer immunotherapy, including co-administering a DEAD-box Helicase 54 (DDX54) inhibitor and a cancer immunotherapy agent to a subject.

The inhibitor may be co-administered simultaneously, separately, or sequentially with the cancer immunotherapy agent.

Still another aspect of the present invention provides a method for treating cancer including administering a DEAD-box Helicase 54 (DDX54) inhibitor and a cancer immunotherapy agent to a subject.

### [Advantageous Effects]

According to the present invention, the DDX54 inhibitor can enhance the efficacy of cancer immunotherapy as a combination target with a cancer immunotherapy agent, and the composition containing the DDX54 inhibitor and the cancer immunotherapy agent inhibits proliferation of cancer, which is a key characteristic thereof, and simultaneously inhibits a signal transduction pathway known as an immune evasion mechanism, and thus may be provided for the prevention or treatment of cancer.

### [Description of Drawings]

FIG. 1 is a diagram illustrating a classification process of a Non-Brisk group with low TIL and a Brisk group with high TIL by inserting tumor-infiltrating lymphocytes (TIL) in lung cancer patient samples with high tumor mutational burden (TMB-H) classified from a TCGA database.
FIG. 2A is a diagram illustrating a low leukocyte ratio including T cells and macrophages in a Non-Brisk group (*: P < 0.05, * *: P < 0.01, ****: P < 0.0001).
FIG. 2B is a diagram illustrating low expression of biomarkers PDCD1 and CD274 for predicting responsiveness to an immune checkpoint inhibitor in a Non-Brisk group (*: P < 0.05).
FIG. 2C is a diagram illustrating that an immune evasion pathway is activated in a Non-Brisk group (**: P < 0.01, ***: P < 0.001).
FIG. 3A is a diagram illustrating ranking of upstream key regulators that most significantly regulate transcription factors showing significant activity in a Non-Brisk group.
FIG. 3B is a diagram illustrating high expression levels of DDX54 in a Non-Brisk group and a TMB-H group (*: P < 0.05).
FIG. 4A is a diagram illustrating that DDX54 is associated with several genes that induce an immune evasion mechanism.
FIG. 4B is a diagram illustrating that transcription factors and target genes regulated by DDX54 induce immune evasion pathways.
FIG. 5A is a diagram illustrating that when DDX54 expression is inhibited in a mouse LLC1 cell line (shDDX54), the expression level of DDX54 mRNA is reduced compared to a control group (PLKO) in which DDX54 expression is not inhibited (**: P < 0.01).
FIG. 5B is a diagram illustrating a result of inhibiting the proliferation of a LLC1 cell line shDDX54 in which DDX54 expression is inhibited.
FIG. 6A is a diagram illustrating that a signal transduction pathway of an immune evasion mechanism has been inhibited in a LLC1 cell line shDDX54 in which DDX54 expression was inhibited, as a DEG analysis result.
FIG. 6B is a diagram illustrating that a signal transduction pathway of an immune evasion mechanism has been inhibited in a LLC1 cell line shDDX54 in which DDX54 expression was inhibited, as a GSEA analysis result.
FIG. 7A is a diagram illustrating quantitative results of reduced expression of cell surface antigen molecules CD248, CD47, CD38, and CD 164 in a LLC1 cell line shDDX54 in which DDX54 expression is inhibited (**: P < 0.01).
FIG. 7B is a diagram illustrating band image results of reduced levels of cell surface antigen molecules CD248, CD47, and CD164 in a LLC1 cell line shDDX54 in which DDX54 expression is inhibited.
FIG. 7C is a diagram illustrating that a JAK-STAT3 signal transduction pathway has been inhibited in a LLC1 cell line shDDX54 in which DDX54 expression was inhibited, as a GSVA analysis result (**: P < 0.01).
FIG. 7D is a diagram illustrating a qRT-PCR result (left) and a western blot result (right) of reduced expression of pJAK1, pSTAT3, p-p65 and MYC in a LLC1 cell line shDDX54 in which DDX54 expression was inhibited.
FIG. 8A is a diagram illustrating results of inhibiting the proliferation of a A549 cell line shDDX54_#2 in which DDX54 expression is inhibited.
FIG. 8B is a diagram illustrating results of reduced MYC expression in A549 cell lines shDDX54_1 and shDDX54_2 and H23 cell lines shDDX54_1 and shDDX54_5 in which DDX54 expression is inhibited.
FIG. 9A is a diagram illustrating results of observing smallest tumor tissues under a combined treatment condition (shDDX54 + α-PD-1 Ab.) in which DDX54 expression was inhibited and a PD-1 inhibitor was treated in tumor model mice.
FIG. 9B is a diagram illustrating that as a result of quantifying the size of tumor tissue, a combined treatment condition (shDDX54 + α-PD-1 Ab.) that inhibited DDX54 expression and was treated with PD-1 inhibitor has inhibited tumor growth compared to other three conditions (***: P < 0.001).
FIG. 9C is a diagram illustrating that as a result of quantifying the weight of tumor tissue, a combined treatment condition (shDDX54 + α-PD-1 Ab.) that inhibited DDX54 expression and was treated with PD-1 inhibitor has inhibited tumor growth compared to other three conditions (***: P < 0.001).
FIG. 9D is a diagram illustrating a result that a combined treatment condition (shDDX54 + α-PD-1 Ab.) that inhibited DDX54 expression and was treated with PD-1 inhibitor for 30 days has the slowest tumor growth compared to other three conditions (***: P < 0.001).
FIG. 9E is a diagram illustrating a result that a combined treatment condition (shDDX54 + α-PD-1 Ab.) that inhibited DDX54 expression and was treated with PD-1 inhibitor for 30 days has significantly high probability of survival compared to other three conditions.
FIG. 10 is a diagram illustrating a distribution by immune cells expressed in tumor tissues of each condition (top) and expression levels according to cell-specific genes by immune cells (bottom).
FIG. 11A is a diagram illustrating that as a result of analyzing immune cell types expressed in tumor tissues of each condition, the tumor infiltration ratios of B cell, DC cell, T cell, and NK cell increased in the combined treatment condition (shDDX54 + α-PD-1 Ab.) that inhibited the DDX54 expression and was treated with the PD-1 inhibitor.
FIG. 11B is a diagram illustrating quantifying UMAP data of FIG. 11A.
FIG. 12 is a diagram illustrating results of flow cytometry showing that CD3 T cell infiltration increased and CD8 T cell infiltration increased in the combined treatment condition (shDDX54 + α-PD-1 Ab.) that inhibited the DDX54 expression and was treated with the PD-1 inhibitor.
FIG. 13 is a diagram illustrating H&E staining results (left) showing that infiltrating immune cells increased compared to the other three conditions in the combined treatment condition (shDDX54 + α-PD-1 Ab.) that inhibited the DDX54 expression and was treated with the PD-1 inhibitor, and a quantification result thereof (right) (*: P < 0.05).
FIG. 14 is a diagram illustrating a schematic diagram of changes in the immune system according to inhibition of DDX54 expression.

### [Best Mode of the Invention]

Unless otherwise contrarily defined, all terms used herein including technological or scientific terms have the same meanings as those generally understood by a person with ordinary skill in the art.

Terms which are defined in a generally used dictionary should be interpreted to have the same meaning as the meaning in the context of the related art, and are not interpreted as an ideal meaning or excessively formal meanings unless otherwise clearly defined in the present application. Further, in describing the present invention, a detailed explanation of known related technologies may be omitted to avoid unnecessarily obscuring the subject matter of the present invention.

The present inventors identified a key regulator DDX54 (DEAD-box Helicase 54) in a patient group with low responsiveness to cancer immunotherapy by applying a tumor mutational burden (TIL), which was approved by the FDA as a biomarker for predicting the responsiveness to cancer immunotherapy, verified that the DDX54 expression was inhibited to exhibit a synergy effect in enhancing a cancer treatment effect of the cancer immunotherapy agent, and then completed the present invention.

In an aspect, the present invention relates to a pharmaceutical composition for the prevention or treatment of cancer including a DEAD-box Helicase 54 (DDX54) inhibitor and a cancer immunotherapy agent.

In the present invention, "DEAD-box Helicase 54 (DDX54)" is a gene encoding a component of the DEAD box protein family. The DEAD box protein, characterized by a conserved motif Asp-Glu-Ala-Asp (DEAD), is considered as RNA helicase and involved in several cellular processes related with modification of RNA secondary structure, such as translation initiation, nuclear and mitochondrial splicing, and ribosome and spliceosome assembly. It is reported that diseases associated with DDX54 include renal hypoplasia, febrile seizures, and the like.

In the present invention, the "inhibitor" means `an agent that inhibits the expression of a target gene' or `an agent that inhibits the activity of a target protein'. The "inhibitor" is interchangeable with "antagonist" or "inhibitor," and "inhibition" may be used interchangeably with "suppression".

The `agent that inhibits the expression of the target gene' may include all types of agents that specifically inhibit or suppress the expression of the target gene, and includes all inhibitors by regulation of a transcriptional process and post-transcriptional translation regulation such as RNAi. For example, the agent may be antisense nucleotide, small interfering RNA (siRNA), small hairpin RNA (shRNA), ribozymes, aptamer, anti-microRNA, micro RNA mimic, nucleases such as Zinc Finger Nucleases (ZFNs) and Transcription activator-like effector nucleases (TALENs), CRISPR-Cas9 system agents (e.g., gRNA, sgRNA), etc., but is not limited thereto.

The antisense nucleotide binds (hybridizes) to a complementary base sequence of DNA, immature mRNA, or mature mRNA, thereby interfering with the flow of genetic information from DNA to protein. The siRNA has a similar operation principle to antisense, but is a double strand consisting of 21 to 25 nucleotides to be linked, and destroys mRNA through an enzyme complex called RNA-induced silencing complexes (RISCs) rather than RNase H. The CRISPR-Cas9 (CRISPR gene scissors) acts as a restriction enzyme that recognizes a specific base sequence and cleaves DNA at the corresponding site.

In the present invention, the target gene is DEAD-box Helicase 54 (DDX54).

The `agent that inhibits the activity of the target protein' may be directly inhibit the activity of the target protein or inhibiting its function by interfering with an interaction with other proteins. For example, the agent may include a compound that specifically binds to protein, a peptide, a peptidomimetic, a substrate analog, an aptamer, and an antibody, but is not limited thereto.

The compound includes any compound that may specifically bind to a target protein to inhibit its activity. The aptamer is a single-stranded DNA or RNA molecule, and may be obtained by isolating oligomers that bind to specific chemical or biological molecules with high affinity and selectivity by an evolutionary method using an oligonucleotide library called systematic evolution of ligands by exponential enrichment (SELEX). The aptamer may specifically bind to a target protein and control the activity of the target protein, for example, block the activity of the target protein through binding. The antibody means a set of antibody protein molecules including one or more complementarity determining regions, a single antibody protein molecule, a binding fragment or a derivative thereof.

In the present invention, the target protein is DEAD-box Helicase 54 (DDX54).

In the present invention, the DDX54 inhibitor may be at least one selected from the group consisting of antisense nucleotide, siRNA, shRNA, ribozyme, aptamer, micro RNA mimic, nuclease, ZFNs, TALENs, gRNA, sgRNA, peptides, peptide mimics, substrate analogs, antibodies or fragments thereof, and vectors expressing the same, which target the DDX54 protein or a gene encoding the same.

In an example of the present invention, the DDX54 inhibitor may use shDDX54 (shDDX54; SHCLNG-NM_028041, Sigma, TRCN0000103746:CCGGTTTATCAGCAGCTCTTA) represented by SEQ ID NO: 1, and may use any agent or means known in the art without limitation, as long as the agent may reduce the DDX54 expression level or activity in cancer cells as the purpose of the present invention.

The present invention may include functional equivalents of the base sequence of shRNA represented by SEQ ID NO: 1. The "functional equivalent" refers to polynucleotide that exhibits substantially the same physiological activity as the polynucleotide represented by the base sequence represented by SEQ ID NO: 1 by having a sequence homology of at least 70% or more, preferably 80% or more, more preferably 90% or more, much more preferably 95% or more with the base sequence represented by SEQ ID NO: 1, as a result of deletion, substitution or insertion of bases. The "% of sequence homology" with the polynucleotide is determined by comparing two optimally arranged sequences with a comparison region, and a part of a polynucleotide sequence in the comparison region may include addition or deletion (i.e., gap) compared to a reference sequence (without including addition or deletion) for an optimal alignment of the two sequences.

According to an example of the present invention, it was confirmed that the expression of DDX54 was up-regulated in cancer immunotherapy agent-resistant cancer cells, and further, it was confirmed that the high expression of DDX54 was associated with the activity of a signal transduction pathway related to immune evasion.

Accordingly, it was confirmed that when cancer cells inhibiting DDX54 was treated with the cancer immunotherapy agent, the immune evasion-related signal transduction pathway and the activity or expression of cell surface antigen molecules were inhibited, and the proliferation of cancer cells was inhibited.

In addition, it was confirmed that tumor growth was significantly inhibited when the DDX54 inhibitor and the cancer immunotherapy agent were treated in combination in tumor model mice.

Therefore, the composition including the DDX54 inhibitor and the cancer immunotherapy agent according to the present invention may be provided for the prevention or treatment of cancer resistant to the cancer immunotherapy agent.

As used in the present invention, the "cancer immunotherapy agent" refers to a therapeutic agent that induces immune cells to selectively attack only cancer cells by injecting an artificial immune protein into the body to stimulate the immune system, unlike conventional anticancer agents that attack cancer itself. The cancer immunotherapy agent may be a drug that recovers or strengthens tumor cognitive ability or destructive ability of the immune system in order to overcome an immune suppression or immune evasion mechanism acquired by cancer cells.

The cancer immunotherapy agent may be divided into an agent used in passive immunotherapy and an agent used in active immunotherapy. The passive immunotherapy includes immune checkpoint inhibitors, immune cell therapies, therapeutic antibodies, etc., and the active immunotherapy includes cancer treatment vaccines, immune-modulating agents, etc., but are not limited thereto.

In the present invention, the cancer immunotherapy agent may be an immune cell therapy, an immune checkpoint inhibitor, or an immune virus therapy.

The immune cell therapy may be selected from the group consisting of a dendritic cell immunotherapy, a LAK cell immunotherapy, a T cell-based immunotherapy, and an NK cell-based immunotherapy, and for example, the T cell-based immunotherapy may be a tumor-infiltrating T cell (TEL), a T cell receptor-expressing T cell (TCR-T), or a chimeric antigen receptor-expressing T cell (CAR-T).

The "immune checkpoint" is also called "immune checkpoint" and refers to a molecule that regulates the activity of immune cells. The immune checkpoint exists for self-tolerance that prevents the immune system from indiscriminately attacking cells. The immune checkpoint may be classified into a stimulatory checkpoint that enhances the immune activity and an inhibitory checkpoint that reduces the immune activity. Some cancers acquire resistance to a cancer immunotherapy agent by targeting an inhibitory immune checkpoint to protect itself from immune responses.

The DDX54 inhibitor of the present invention may reduce the resistance of cancer cells to an immune anticancer agent, but is not limited thereto, and preferably may mean reducing the resistance to the immune checkpoint inhibitor.

Therefore, the cancer immunotherapy agent of the present invention is not limited thereto, but may preferably be an immune checkpoint inhibitor.

The immune checkpoint inhibitor is a drug that activates immune cells to attack cancer cells by blocking the activity of a target immune checkpoint protein involved in immune cell suppression.

Immune checkpoints that may be targeted by the immune checkpoint inhibitor include PD-1, PD-L1, CD80, CD86, CTLA4, KIRs, BTLA, TNFRSF4, GITRL, 4-1BB, VTCN1, TIM-3, TIGIT, VISTA, etc. Accordingly, the immune checkpoint inhibitor may be at least one selected from the group consisting of a PD-1 inhibitor, a PDL-1 inhibitor, a PD-L2 inhibitor, a CTLA-4 inhibitor, a LAG-3 inhibitor, a TIM-3 inhibitor, a CEACAM1 inhibitor, a CD27 inhibitor, a CD28 inhibitor, a CD70 inhibitor, a CD80 inhibitor, a CD86 inhibitor, a CD137 inhibitor, a CD276 inhibitor, a CTLA4 inhibitor, a KIRs inhibitor, a LAG3 inhibitor, a TNFRSF4 inhibitor, a GITR inhibitor, a GITRL inhibitor, a 4-1BBL inhibitor, a A2AR inhibitor, a VTCN1 inhibitor, a BTLA inhibitor, a IDO inhibitor, a TIM-3 inhibitor, a VISTA inhibitor, a KLRA inhibitor, and combinations thereof.

In an example of the present invention, a PD-1 inhibitor (BioXCell, InVivoMAb anti-mouse PD-1, Cat#BE0273) was used, but any immune checkpoint inhibitor that may be used clinically may be used without limitation.

The composition including the DDX54 inhibitor and the cancer immunotherapy agent according to the present invention, which reduces the resistance of the cancer cells to the cancer immunotherapy agent, may inhibit the activity of at least one signal transduction pathway selected from the group consisting of JAK-STAT3, MYC, STEMNESS, EMT, and WNT, known as an immune evasion signal transduction pathway.

According to an example of the present invention, when comparing a case where DDX54 expression was inhibited through tumor model mice, a case where DDX54 expression was not inhibited and the PD-1 inhibitor was treated, a case where DDX54 expression was inhibited and the PD-1 inhibitor was treated in combination, and a case where both the DDX54 and the PD-1 inhibitor were not treated, it was confirmed that in tumor tissues under combination treatment conditions that inhibited DDX54 expression and was treated with the PD-1 inhibitor, infiltrated immune cells such as monocytes and macrophages (Mono/Mac), B cells, NK cells, dendritic cells (DC), CD8 T cells, and CD4 T cells were significantly increased compared to other three conditions.

As the result, it was confirmed that high expression of DDX54 was positively correlated with low efficacy of the cancer immunotherapy agent in a cancer immunotherapy group with low tumor infiltrating lymphocytes despite a high tumor mutational burden (THB).

Therefore, the composition for the prevention or treatment of cancer according to the present invention may include the DDX54 inhibitor and the cancer immunotherapy agent to increase tumor-infiltrating lymphocytes (TIL), and may mean that the number of immune cells infiltrated in the tumor increases and a tumor microenvironment is activated with anti-tumor immunity.

The cancer may include any cancer as long as the composition according to the present invention achieves the intended effect, and may be solid cancer.

For example, the cancer may be at least one selected from the group consisting of breast cancer, head and neck cancer, uterine cancer, brain cancer, skin cancer, kidney cancer, lung cancer, colorectal cancer, prostate cancer, liver cancer, bladder cancer, kidney cancer, pancreatic cancer, thyroid cancer, esophageal cancer, eye cancer, stomach cancer, ovarian cancer, cervical cancer, gallbladder cancer, bile duct cancer, and osteosarcoma, and in an example of the present invention, the therapeutic efficacy of the composition according to the present invention was presented for lung cancer.

In the present invention, the pharmaceutical composition means a composition containing the DDX54 inhibitor and the cancer immunotherapy agent as active ingredients sufficient to achieve a predetermined efficacy or activity. The active ingredient may be administered in a pharmaceutically effective amount, and the effective dose level may be determined depending on the type, age, and sex of a subject, sensitivity to a drug, a treatment period, drugs used simultaneously, and other medical factors.

The pharmaceutical composition may be in the form of capsules, tablets, granules, injections, ointments, powders or beverages, and the pharmaceutical composition may target humans. The pharmaceutical composition is not limited thereto, but may be formulated and used in the form of oral formulations, such as powders, granules, capsules, tablets, aqueous suspensions, etc., external preparations, suppositories, and sterile injectable solutions according to a conventional method, respectively.

The pharmaceutical composition according to the present invention may include a pharmaceutically acceptable carrier. The pharmaceutically acceptable carrier may be used with a binder, a lubricant, a disintegrant, an excipient, a solubilizer, a dispersant, a stabilizer, a suspending agent, a pigment, a flavoring, and the like during oral administration, may be mixed and used with a buffering agent, a preservative, a painless agent, a solubilizer, an isotonic agent, a stabilizer, and the like in the case of injections, and may be used with a base, an excipient, a lubricant, and a preservative, and the like in the case of topical administration. The formulations of the pharmaceutical composition of the present invention may be prepared variously in combination with the pharmaceutically acceptable carrier described above.

For example, for oral administration, the pharmaceutical composition may be formulated in the form of tablets, troches, capsules, elixirs, suspensions, syrups, wafers, etc., and for injections, the pharmaceutical composition may be formulated into a single dose ampoule or a multiple dose form. In addition, the pharmaceutical composition may be formulated into solutions, suspensions, tablets, capsules, sustained release agents, and the like.

Meanwhile, examples of the carrier, the excipient, and the diluent suitable for the formulations may be used with lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, mineral oils, or the like. In addition, the pharmaceutical composition may further include fillers, anti-coagulating agents, lubricants, wetting agents, flavorings, emulsifiers, preservatives, and the like.

The route of administration of the pharmaceutical composition according to the present invention is not limited thereto, but includes oral, intravenous, intramuscular, intraarterial, intramedullary, intrathecal, intracardiac, transdermal, subcutaneous, intraperitoneal, intranasal, intestinal, topical, sublingual or rectal route. The "parenteral" includes subcutaneous, intradermal, intravenous, intramuscular, intraarticular, intrasynovial, intrasternal, intrathecal, intralesional and intracranial injection or infusion techniques. The pharmaceutical composition according to the present invention may also be administered in the form of a suppository for rectal administration.

The pharmaceutical composition according to the present invention may be variously changed according to various factors, including the activity of a specific active ingredient used, age, body weight, general health, sex, diet, administration time, administration route, excretion rate, drug combination, and the severity of a specific disease to be prevented or treated. The dose of the pharmaceutical composition varies depending on the condition, body weight, degree of a disease, drug form, and administration route and period of a patient, but may be appropriately selected by those skilled in the art. Preferably, the pharmaceutical composition may be administered with an amount capable of obtaining a maximum effect with a minimum amount without side effects in consideration of all the factors, and may be repeatedly administered in an effective amount of more preferably 1 to 10000 µg/weight kg/day, and much more preferably 10 to 1000 mg/weight kg/day several times a day. The dose does not limit the scope of the present invention in any aspect.

The pharmaceutical composition of the present invention may be used alone or in combination with surgery, radiation therapy, hormone therapy, chemotherapy, and methods of using biological response modifiers, for prevention or treatment of target indications.

In another aspect, the present invention relates to a composition for enhancing the efficacy of cancer immunotherapy including a DEAD-box Helicase 54 (DDX54) inhibitor.

As described above, in an example of the present invention, when DDX54 expression is inhibited in cancer cells and the cancer immunotherapy agent is administered, it was verified that the resistance of cancer cells to the cancer immunotherapy agent was reduced, and thus the number of immune cells infiltrated in the tumor increased, and the tumor microenvironment was activated with anti-tumor immunity.

Therefore, the composition for enhancing the efficacy of the cancer immunotherapy may enhance the efficacy of cancer immunotherapy by reducing the resistance of cancer to the cancer immunotherapy agent and enhancing anti-tumor immune responses. That is, the DDX54 inhibitor may be used as an adjuvant to enhance the cancer immunotherapy efficacy of the cancer immunotherapy agent.

Since the DDX54 inhibitor and the cancer immunotherapy agent are as described above, description of duplicated contents thereof will be omitted.

In yet another aspect, the present invention relates to a method for enhancing the efficacy of cancer immunotherapy, including co-administering a DEAD-box Helicase 54 (DDX54) inhibitor and a cancer immunotherapy agent to a subject.

Further, the present invention relates to a method for treating cancer including administering a DEAD-box Helicase 54 (DDX54) inhibitor and a cancer immunotherapy agent to a subject.

For example, the method may include measuring the expression or activity level of a DDX54 gene or a protein thereof in a sample isolated from a subject; and
co-administering a DDX54 inhibitor and a cancer immunotherapy agent, when the expression or activity level of the DDX54 gene or the protein thereof is increased compared to a normal control group or a comparative group sample exhibiting the responsiveness to the cancer immunotherapy agent.

For example, the method may include measuring the level of tumor infiltrating lymphocytes (TILs) in a sample isolated from a subject; and
co-administering a DDX54 inhibitor and a cancer immunotherapy agent, when the level of tumor infiltrating lymphocytes (TILs) is lower than a normal control group or a comparative group sample exhibiting the responsiveness to the cancer immunotherapy agent.

The inhibitor may be co-administered simultaneously, separately, or sequentially with the cancer immunotherapy agent. The administration order may vary depending on various factors and may be determined by those skilled in the art.

The 'subject' is a target having cancer disease, and may be a subject that has resistance or low responsiveness to the cancer immunotherapy agent. The subject also means mammals such as horses, sheep, monkeys, pigs, goats, dogs, etc., including humans, but preferably humans.

Since the DDX54 inhibitor and the cancer immunotherapy agent are as described above, description of duplicated contents thereof will be omitted.

In yet another aspect, the present invention relates to a combination agent for enhancing the activity of an immune checkpoint inhibitor including a DEAD-box Helicase 54 (DDX54) inhibitor and an immune checkpoint inhibitor.

The DDX54 inhibitor and the immune checkpoint inhibitor are as described above.

According to an example of the present invention, it was confirmed that even in a cancer type that is resistant to a cancer immunotherapy agent, when the DDX54 expression is inhibited and the PD-1 inhibitor is treated in combination in a cancer cell line, immune cells infiltrated in the tumor increased and the tumor microenvironment was activated with anti-tumor immunity, so that the DDX54 inhibitor may enhance the activity of the immune checkpoint inhibitor.

### [Modes of the Invention]

Hereinafter, the present invention will be described in more detail with reference to Examples. These Examples are only illustrative for the present invention, and it will not be understood that the scope of the present invention is limited by these Examples.

### Example 1: Profiling of key regulators in low cancer immunotherapy group

### 1-1. Selection of Non-Brisk group with low responsiveness to cancer immunotherapy

Samples containing mutation data and tumor-infiltrating lymphocytes (TIL) data from the Cancer Genome Atlas (TCGA) database were obtained, and lung cancer patient samples with a high tumor mutation burden (TMB > 10 per megabase) were preferentially selected. TMB calculation was performed by dividing the total mutation frequency accumulated in the genome by the length of a typical human exon of 38 Mb.

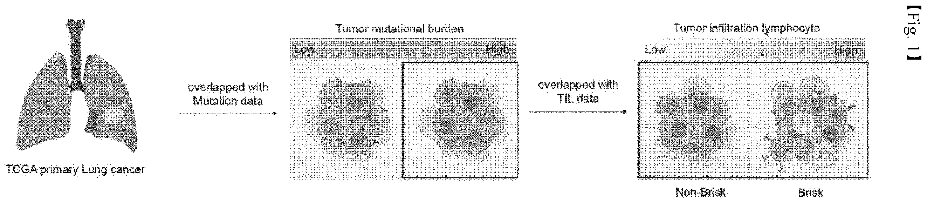

The selected samples were classified into a Non-Brisk group with a high tumor mutation burden (TMB-H) and low tumor-infiltrating lymphocytes and a Brisk group with high tumor-infiltrating lymphocytes by reflecting tumor infiltrating lymphocyte data (FIG. 1).

Through data analysis of the classified Non-Brisk group, gene signatures were selected as genes showing significant differences from the Brisk group using differentially expressed genes (DEGs) analysis and gene set enrichment analysis (GSEA). The DEGs were obtained using the R package DESeq2 and selected based on the criteria of log2 fold change > 1 and p-value < 0.05. The GSEA used version v4.2.3 and utilized Fragments Per Kilobase of exon per Million (FPKM) normalized expression data.

### 1-2. Resistance to cancer immunotherapy agent in Non-Brisk group

It was confirmed that a Non-Brisk group, which had a high tumor mutational burden but low tumor-infiltrating lymphocytes, had less immune cell infiltration.

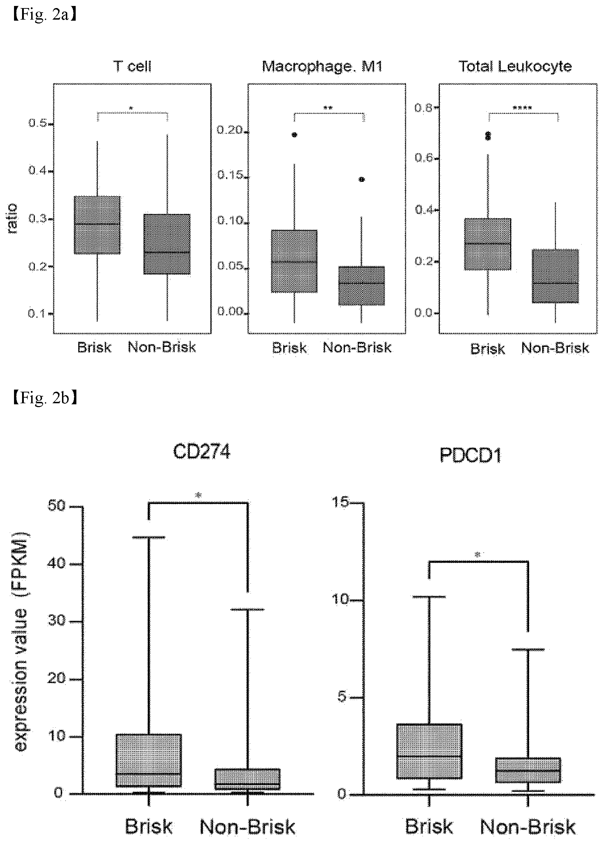

As a result of analyzing the Non-Brisk group using a CIBERSORT analysis tool, which calculated a ratio of cell types from gene expression data, as shown in FIG. 2A, it may be confirmed that the Non-Brisk group has a lower ratio of total leukocytes than the Brisk group, and even among the leukocytes, the ratios of T cells and M1 Macrophages are low.

The Non-Brisk group, which exhibited these low tumor-infiltrating lymphocytes, was identified as having an activated immune evasion pathway and resistance to immune checkpoint inhibitors.

Referring to FIG. 2B, it may be confirmed that the expression values of PDCD 1 (PD-1) and CD274 (PD-L1), which are biomarkers for predicting the responsiveness to immune checkpoint inhibitors, are lower in the Non-Brisk group than the Brisk group.

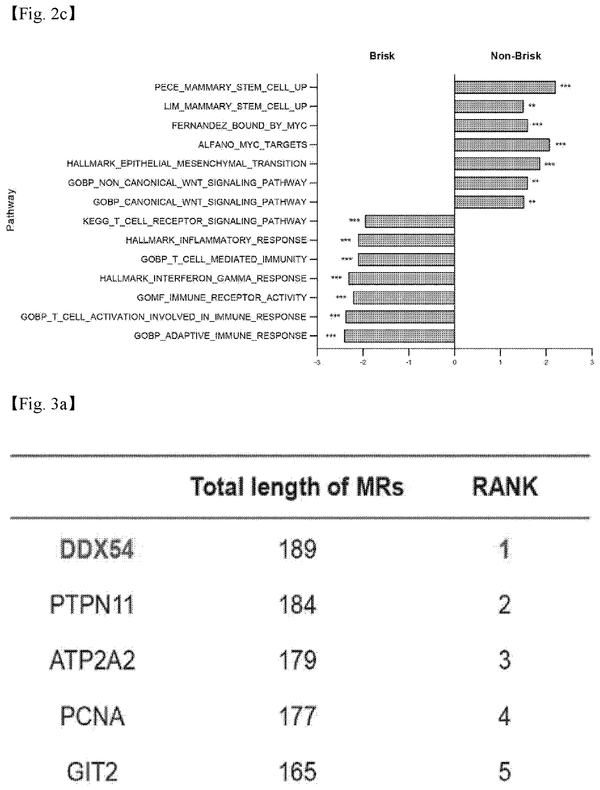

In addition, referring to FIG. 2C, as the GSEA analysis result, it may be seen that previously known immune evasion pathways, such as a STEM-related pathway, a MYC-related pathway, a WNT signaling pathway, and a EMT pathway, are activated (blue bars) in the Non-Brisk group.

### 1-3. Identification of key regulators in Non-Brisk group

A gene regulatory network was obtained using an algorithm for the reconstruction of gene regulatory networks (ARACNe). In this process, FPKM normalized expression data and a list of 1772 transcription factors of lung cancer were used, and mutual information p-value < 10⁻⁸, bootstrap = 100 was executed with default parameters to derive a significant relationship between transcription factors and target genes.

Thereafter, the activity of transcription factors was calculated with a Virtual Inference of Protein activity by Enriched Regulon analysis (VIPER) algorithm, using the gene regulatory network and gene signature list obtained through the previous analysis. Finally, a key upstream regulator that most significantly regulates transcription factors in the Non-Brisk group was identified and selected as a target through a Driver-gene Inference by Genetical-Genomic Information Theory (DIGGIT) algorithm by reflecting copy number variation (CNV) data.

Through the data analysis process, significant upstream regulators were discovered in the Non-Brisk group, which had a high tumor mutation burden and no immune cell infiltration.

Referring to FIG. 3A, which shows the ranking of upstream regulators according to the data analysis result, DDX54 ranked highest as the key upstream regulator that most significantly regulated transcription factors showing significant activity in the Non-Brisk group.

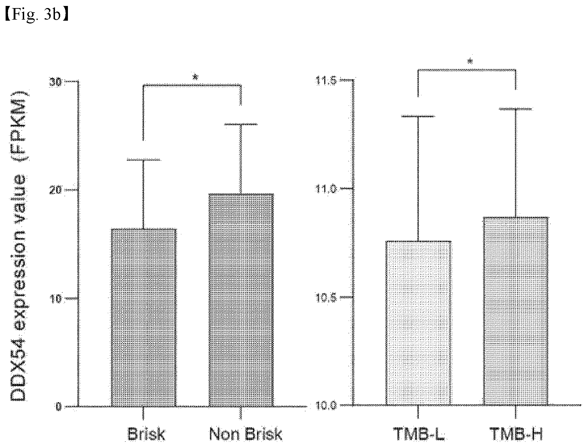

As a result of comparing the DDX54 gene expression levels between the Brisk and Non-Brisk groups, and between the low and high TMB groups, from the data classified from the TCGA data, as shown in FIG. 3B, it may be confirmed that the expression level of DDX54 in the Non-brisk group significantly increased compared to the Brisk group, and it may also be confirmed that the expression level of DDX54 increased in the TMB-H group with a high tumor mutation burden.

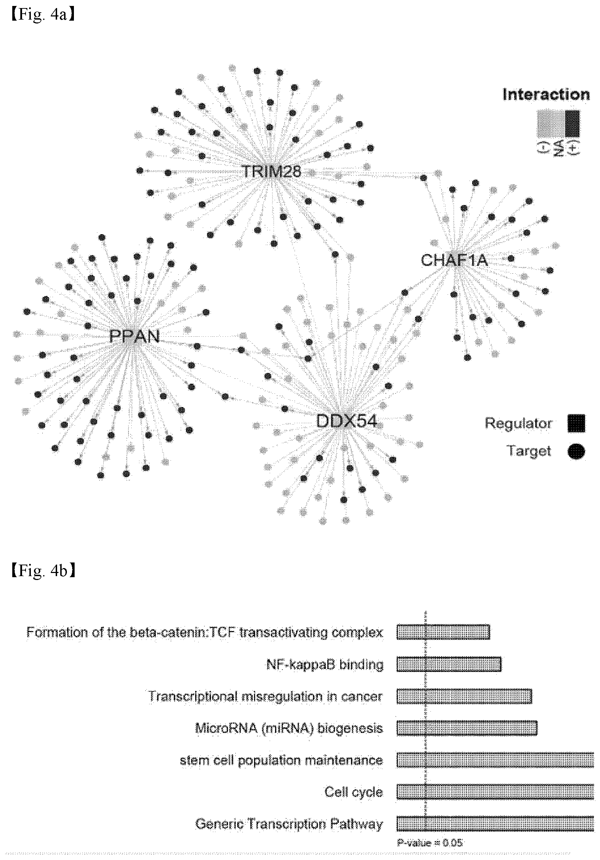

It was confirmed through the gene regulatory network that the DDX54 was associated with several genes that induced an immune evasion mechanism (FIG. 4A). In addition, as a result of performing Gene ontology (GO) analysis of transcription factors and target genes obtained through the regulatory network of DDX54, as shown in FIG. 4B, it was confirmed that the transcription factors and the target genes regulated by DDX54 had the function of activating the immune evasion pathway.

Based on the results, DDX54 was selected as a key regulator of a cancer immunotherapy group to be expected to show low responsiveness to an immune anticancer agent that activated the immune evasion pathway, and the following experiment was conducted.

### Example 2: Correlation between signal transduction pathway of immune evasion mechanism and DDX54 expression

### 2-1. Inhibition of DDX54 expression in mouse cell line

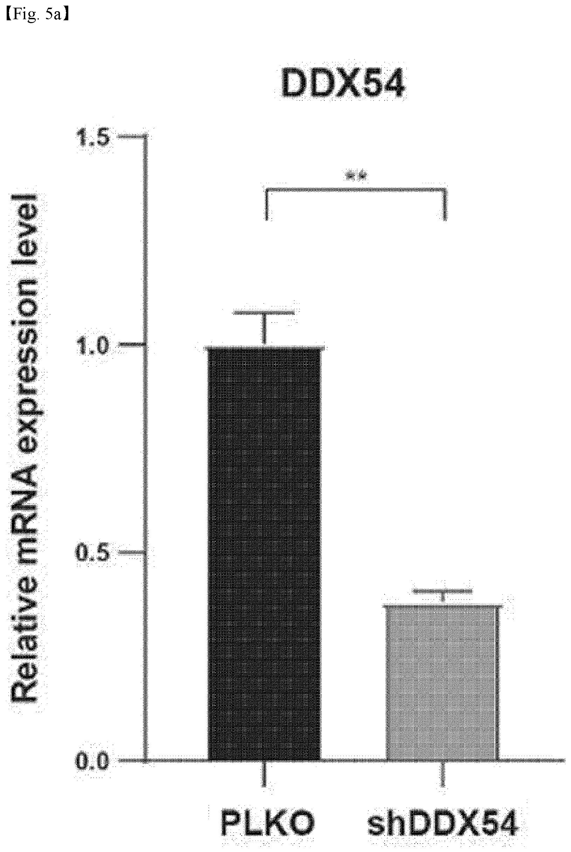

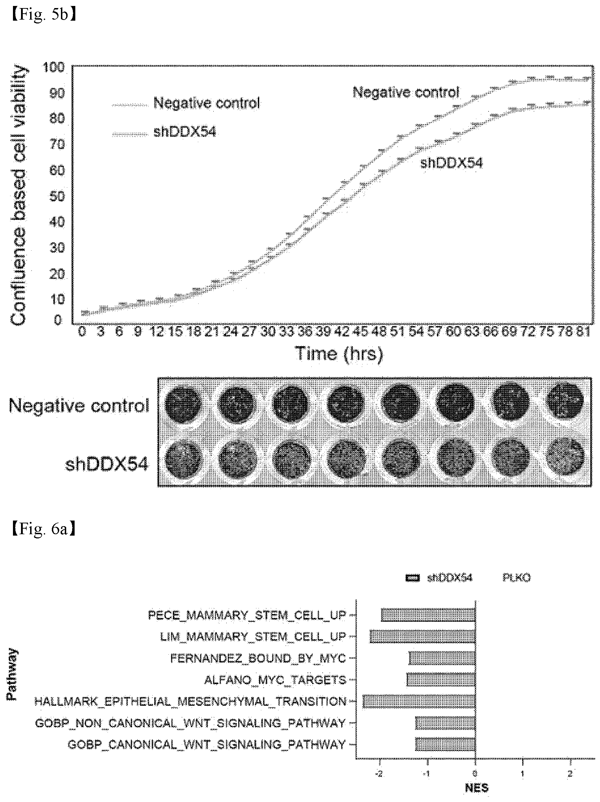

FIG. 5 illustrated results of analyzing cell growth ability and a signal transduction pathway of an immune evasion mechanism when DDX54 expression was inhibited in a LLC1 cell line derived from C57BL/6 mice.

First, the LLC 1 cell line was treated with shDDX54 (SHCLNG-NM_028041, Sigma, TRCN0000103746:CCGGTTTATCAGCAGCTCTTA; SEQ ID NO: 1) and qRT-PCR was performed to confirm that DDX54 expression was significantly inhibited (FIG. 5A), and then a cell growth ability experiment was conducted. As a result, as seen in a cell line survival curve graph at the top of FIG. 5B and a cell culture image at the bottom of FIG. 5B, it may be confirmed that the proliferation of the cell line treated with shDDX54 was reduced compared to a negative control group (gray, N.C).

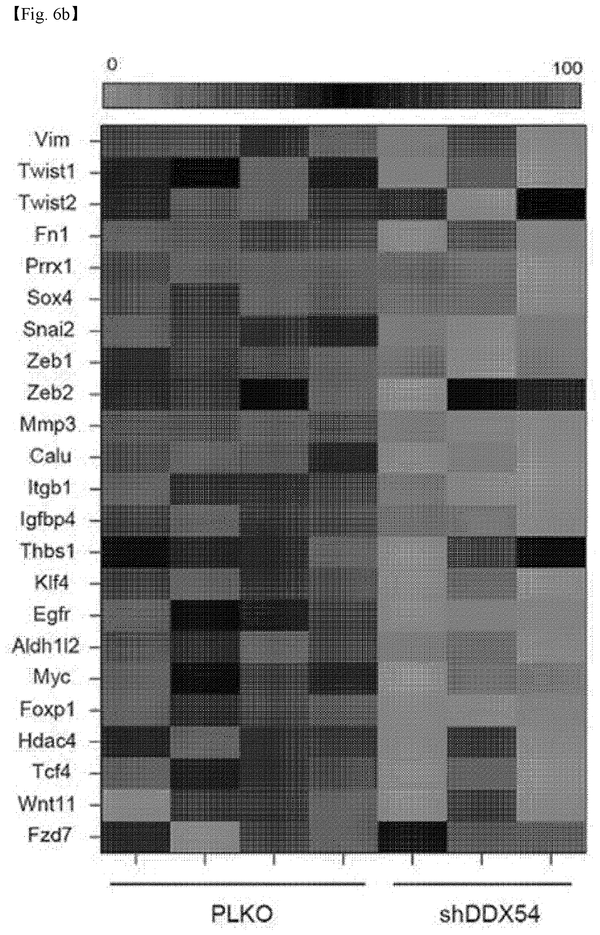

Next, for a shDDX54 treated experimental group (shDDX54) and a shDDX54 untreated control group (PLKO), results of performing DEG analysis and GSEA analysis using data obtained through Bulk RNA sequencing were shown in FIG. 6.

Referring to FIGS. 6A and 6B, it may be seen that in the shDDX54 experimental group that inhibited DDX54 expression, immune evasion-related signal transduction pathways MYC, STEMNESS, EMT, and WNT are inhibited. The different signal transduction pathway activities between the shDDX54 experimental group and the PLKO control group may also be confirmed in FIG. 6B, where the data is converted into a cluster heatmap.

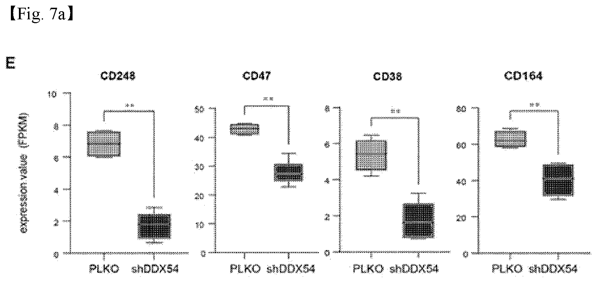

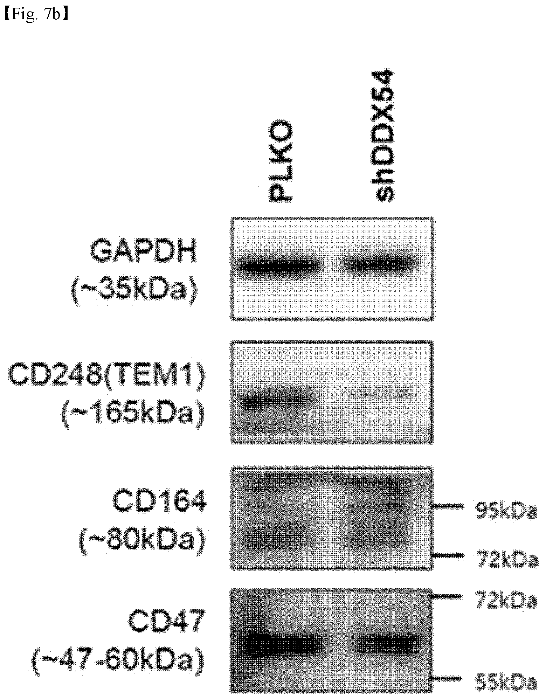

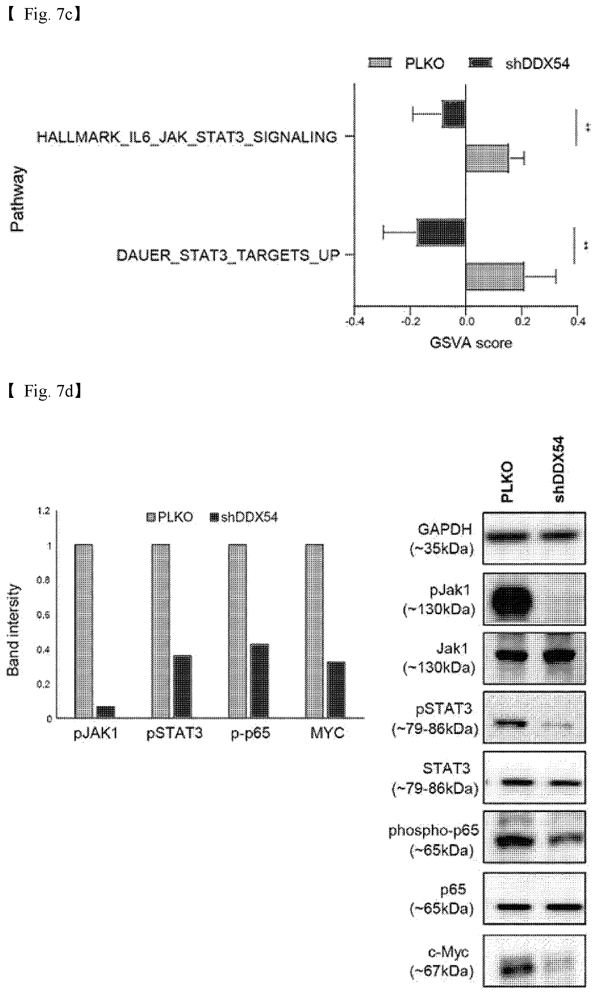

Further, FIG. 7 illustrated results of performing Western blot for cluster of differentiation (CD) molecules CD38, CD47, CD164, and CD248, which were known to contribute to immune evasion.

Referring to FIG. 7A, it may be confirmed that the expression of CD38, CD47, CD164, and CD248 molecules is inhibited in the shDDX54 experimental group that inhibited DDX54 expression, compared to the control group (PLKO). In addition, as a result of confirming the protein amounts of CD47, CD 164, and CD248 decreased by DDX54 inhibition through Western blot, as shown in FIG. 7B, it was confirmed that the expression of CD47, CD164, and CD248 molecules was decreased in the shDDX54 experimental group.

Additionally, it was confirmed that the JAK-STAT3 signaling pathway was inhibited in the shDDX54 experimental group that inhibited DDX54 expression from the previous GSVA analysis results (FIG. 7C), and the results of performing Western blot were shown in FIG. 7D. When describing the bands on the right and the quantitative values on the left of FIG. 7D, it may be confirmed that the expression levels of p65 and MYC genes present in the JAK-STAT3 pathway and sub-pathways thereof were reduced in the shDDX54 experimental group that inhibited DDX54 expression.

Through the results of FIGS. 5 to 7, it was confirmed that when DDX54 was inhibited in cancer cells, the proliferation was reduced at the cell line level, a signal transduction pathway known as the immune evasion mechanism was inhibited, and molecules related to immune evasion were inhibited. Accordingly, it was confirmed that DDX54, discovered in cancer types resistant to the cancer immunotherapy agent, may become a key regulator of cancer immunotherapy.

### 2-2. Inhibition of DDX54 expression in human lung cancer cell lines

The effects of DDX54 regulation were analyzed using human lung cancer cell lines A549 and H23.

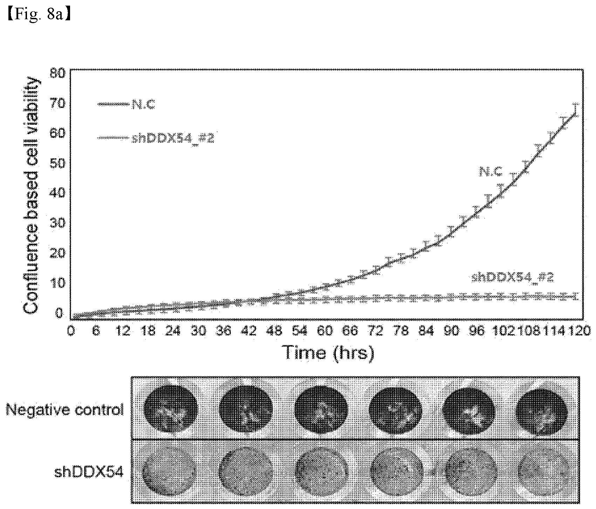

First, referring to FIG. 8A illustrating a growth ability result of an A549 cell line which was treated with shDDX54 (SHCLNG-NM_028041, Sigma, TRCN0000103746:CCGGTTTATCAGCAGCTCTTA; SEQ ID NO: 1) to inhibit DDX54 expression, as shown in a cell viability curve graph of the top and a cell culture image of the bottom, it was confirm that the cell proliferation was significantly inhibited in the shDDX54 treated experimental group (shDDX5_#2) compared to the shDDX54 untreated control group (N.C).

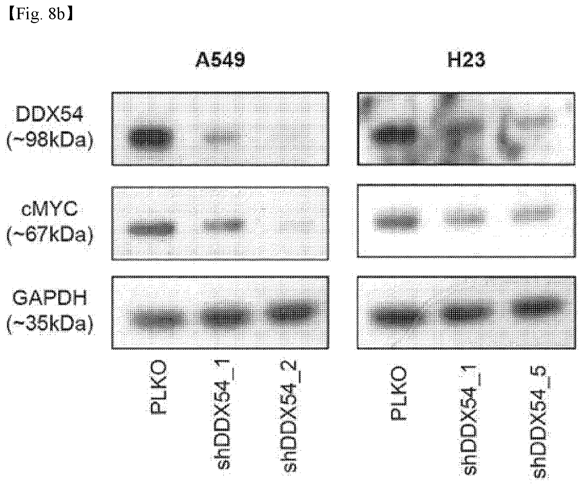

In addition, referring to FIG. 8B, it was shown that in both the A549 cell line, where DDX54 expression was inhibited by shDDX54 treatment, and the H23 cell line, where DDX54 expression was inhibited by shDDX54 treatment, the MYC signal transduction pathway was inhibited.

As these results, it may be expected that the inhibition of DDX54 not only has the effect of inhibiting the proliferation of cancer cells, but also reduces the resistance of cancer cells to the cancer immunotherapy agent by inhibiting the immune evasion mechanism.

### Example 3: Inhibition of DDX54 expression and combined treatment with immune checkpoint inhibitor

Whether DDX54 inhibition could reduce resistance to a cancer immunotherapy agent and enhance the efficacy of cancer immunotherapy was confirmed using C57BL/6 mice injected with a LLC1 cell line in which the DDX54 expression was inhibited.

### 3-1, Inhibitory effect on tumor growth in tumor model mice

The tumor growth and changes in tumor tissue were observed for 30 days under a condition (shDDX54 + α-PD-1 Ab.) of injecting C57BL/6 mice with a LLC1 cell line which was treated with shDDX54 (SHCLNG-NM_028041, Sigma, TRCN0000103746:CCGGTTTATCAGCAGCTCTTA; SEQ ID NO: 1) to inhibit DDX54 expression and co-administering a PD-1 inhibitor (BioXCell, InVivoMAb anti-mouse PD-1, Cat#BE0273) as an immune checkpoint inhibitor, a condition (shDDX54 + Iso Ab.) without co-administering the PD-1 inhibitor, a condition (Wild type + Iso Ab.) without treating both shDDX54 and PD-1 inhibitor, and a condition (Wild type + α-PD-1 Ab.) of treating only the PD-1 inhibitor without treating shDDX54.

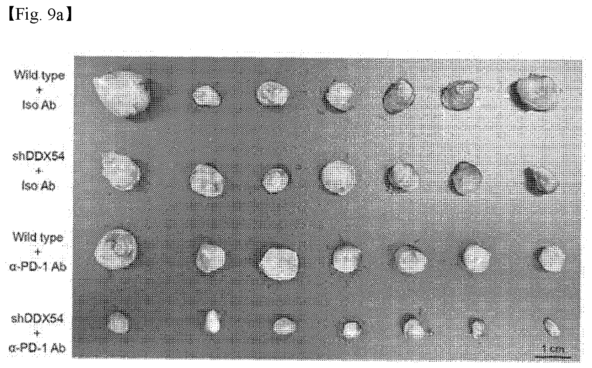

FIG. 9A shows images of tumor tissues extracted from mice under each condition. Referring to FIG. 9A, it may be clearly seen with the naked eye that the tumor size under the shDDX54 and PD-1 inhibitor treated condition (shDDX54 + α-PD-1 Ab.) was smaller than that under other three conditions without combined treatment.

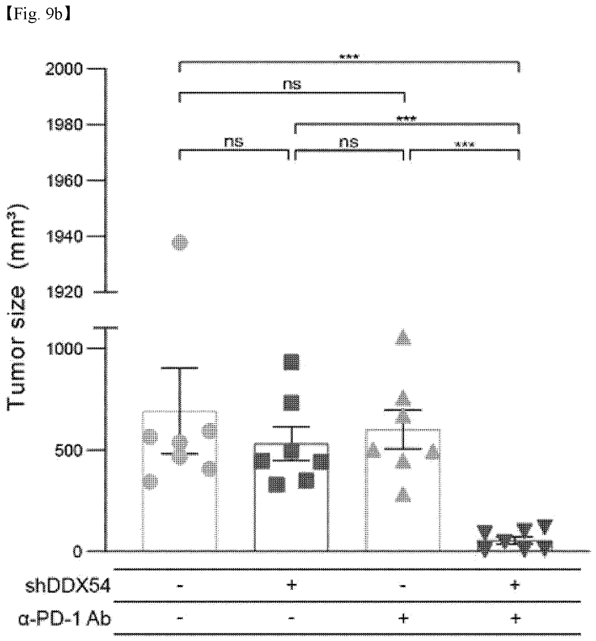

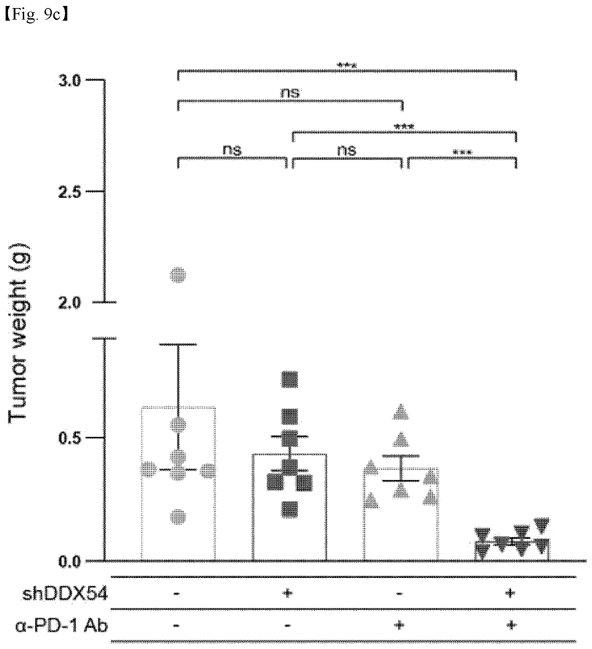

In addition, the results of quantifying and comparing the weights and sizes of tumor tissues extracted from mice under each condition were shown in FIGS. 9B and 9C. Referring to FIGS. 9B and 9C, it was confirmed that there was no significant difference (ns) between the other three conditions without combined treatment, while the shDDX54 and PD-1 inhibitor treated condition (shDDX54 + α-PD-1 Ab.) showed a significant difference (***) from the three conditions above, and both the weight and size of the tumor tissue were the lowest, which was similar to the results confirmed with the naked eye.

Specifically, the condition (shDDX54 + α-PD-1 Ab.) of inhibiting the DDX54 expression and treating the PD-1 inhibitor showed a 4.66-fold decrease in tumor weight and an 11.09-fold decrease in tumor size compared to the condition (Wild type + α-PD-1 Ab.) of only treating the PD-1 inhibitor without inhibiting the DDX54 expression.

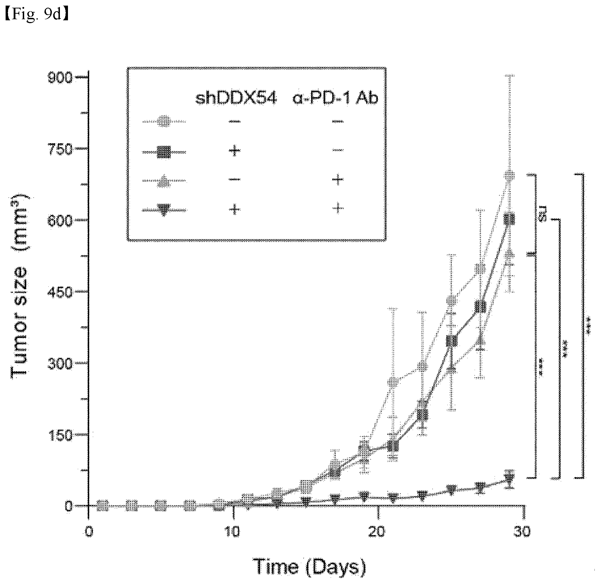

The results of analyzing the tumor growth rate by measuring the size of tumor tissue at two-day intervals while observing for 30 days were shown in FIG. 9D. Referring to FIG. 9D, it may be confirmed that the tumor growth rate under the condition (shDDX54 + α-PD-1 Ab.) of inhibiting the DDX54 expression and treating the PD-1 inhibitor was significantly slower than that of the other three conditions without combined treatment.

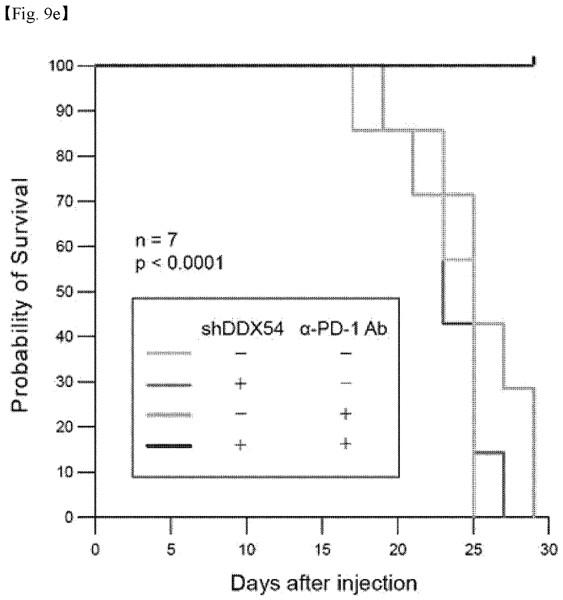

In addition, as the result of analyzing the probability of survival of mice under each condition, referring to FIG. 9E, it may be confirmed that the probability of survival was significantly high under the condition (shDDX54 + α-PD-1 Ab.) of inhibiting the DDX54 expression and treating the PD-1 inhibitor.

From the results illustrated in FIG. 9, it was confirmed that the anticancer efficacy against cancer cells showing resistance to cancer immunotherapy may be enhanced by inhibiting the expression of DDX54, which was discovered as a key regulator in a cancer type showing resistance to a cancer immunotherapy agent.

### 3-2. Enhanced effect of tumor-infiltrating immune cells in tumor model mice

As a result of classifying immune cells by performing RNA sequencing based on cell-specific genes on immune cells discovered in tumor tissues under each condition of Example 3-1, it was confirmed that when the DDX54 expression inhibition and the PD-1 inhibitor were combined, the number of immune cells infiltrated in the tumor increased and the tumor microenvironment was activated with anti-tumor immunity.

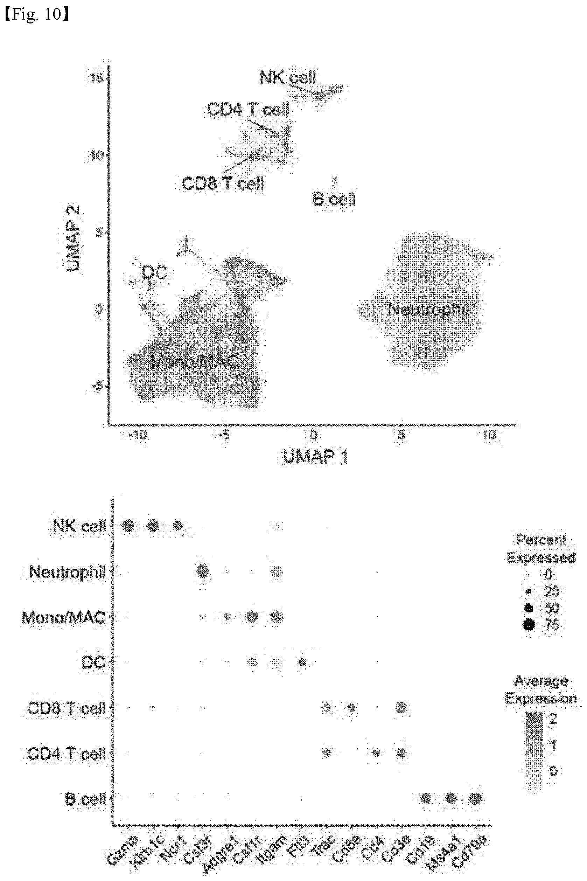

Specifically, after preprocessing data derived from RNA sequencing, Uniform Manifold Approximation and Projection (UMAP) was executed and the results of immune cell distribution according to the shDDX54 and PD-1 inhibitor condition using cell-specific genes were shown in FIG. 10.

Referring to the UMAP results in the upper image of FIG. 10, as a result of classifying cell types of cell clusters obtained from the data, it may be confirmed that immune cells such as NK cells, neutrophils, monocytes and macrophages (Mono/Mac), B cells, NK cells, dendritic cells (DC), CD8 T cells, and CD4 T cells are differentially distributed.

The expression levels of the immune cells were represented as a dot plot (bottom image) consisting of data points according to cell-specific gene biomarkers expressed within the cells, respectively.

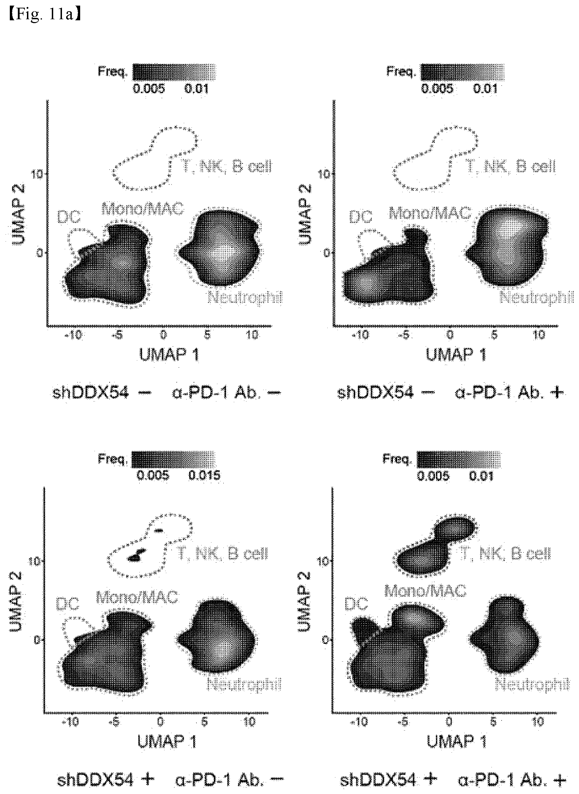

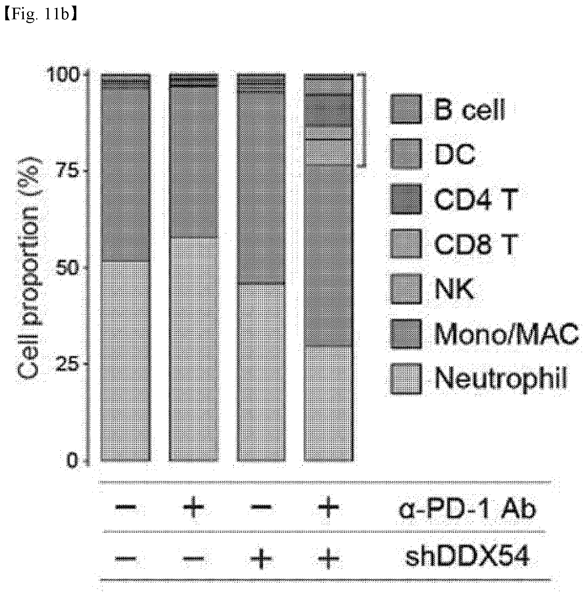

As a result of analyzing immune cell types expressed in tissues under each condition by performing UMAP, as shown in FIG. 11A, it was confirmed that infiltration ratios of B cell, DC cell, T cell, and NK cell increased in the tumor tissues under the combined treatment condition (shDDX54 + α-PD-1 Ab.) that inhibited the DDX54 expression and treated the PD-1 inhibitor. Referring to FIG. 11B of quantifying the data, it may be seen that monocytes and macrophages (Mono/Mac), B cells, NK cells, dendritic cells (DC), CD8 T cells, and CD4 T cells are clearly increased.

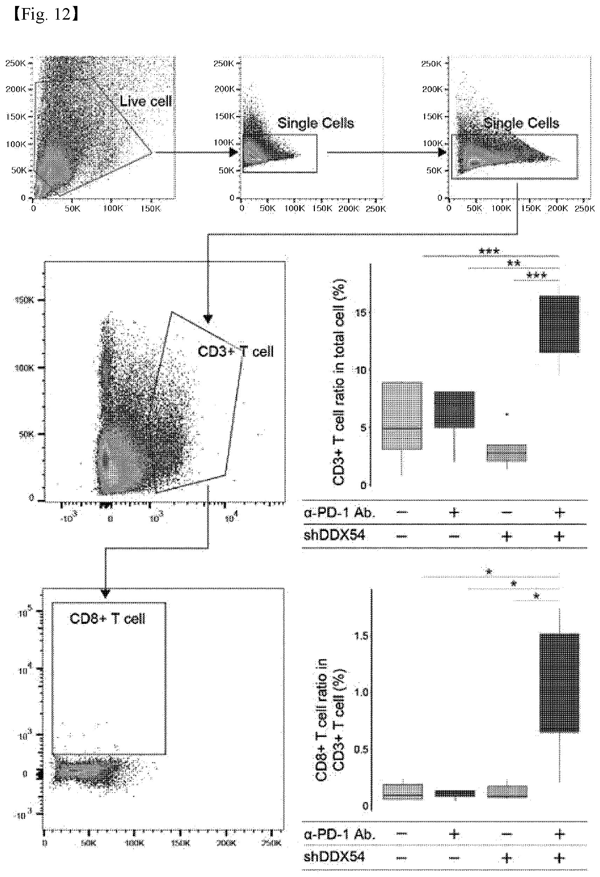

In addition, as a result of analyzing increased infiltrating-immune cells through Flow cytometry in the tumor tissues under the combined treatment condition (shDDX54 + α-PD-1 Ab.) that inhibited the DDX54 expression and treated the PD-1 inhibitor, as shown in FIG. 12, the increased infiltration of CD8 T cells was confirmed.

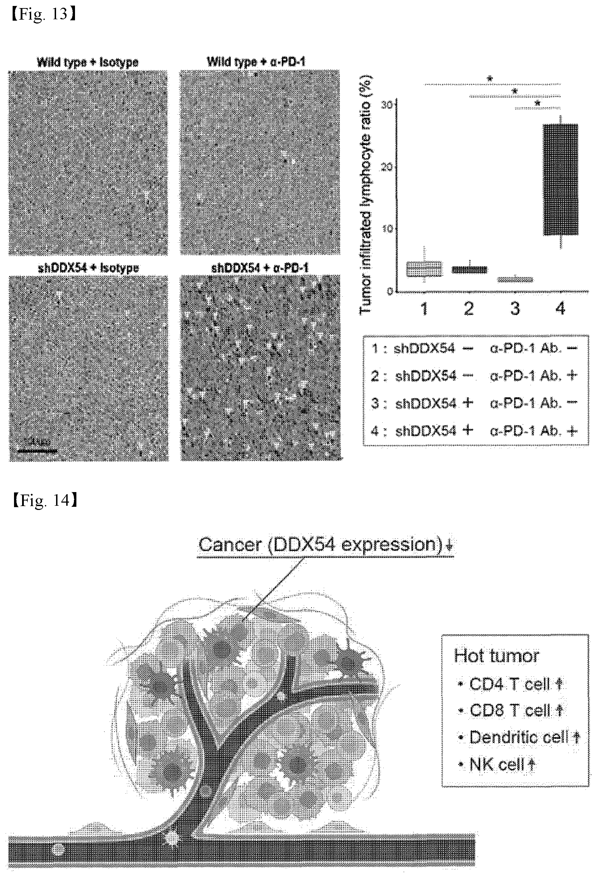

In addition, the results of performing hematoxylin & eosin staining (H&E staining) on the tissue under each condition were shown in FIG. 13. Referring to the staining images and the quantification graph of FIG. 13, it may be confirmed that the infiltrated immune cells significantly increased in the tumor tissue under the combined treatment condition (shDDX54 + α-PD-1 Ab.) that inhibited the DDX54 expression and treated the PD-1 inhibitor compared to the other three conditions.

Through Example 3, it was confirmed that even in cancer types resistant to the cancer immunotherapy agent, when DDX54 expression was inhibited and the PD-1 inhibitor was co-treated in cancer cell lines, immune cells infiltrated in the tumor increased and the tumor microenvironment was activated with anti-tumor immunity.

## Claims

1. A pharmaceutical composition for the prevention or treatment of cancer comprising a DEAD-box Helicase 54 (DDX54) inhibitor and a cancer immunotherapy agent.

2. The pharmaceutical composition for the prevention or treatment of cancer of claim 1, wherein the DDX54 inhibitor is at least one selected from the group consisting of antisense nucleotide, siRNA, shRNA, ribozyme, aptamer, micro RNA mimic, nuclease, ZFNs, TALENs, gRNA, sgRNA, peptides, peptide mimics, substrate analogs, antibodies or fragments thereof, and vectors expressing the same, which target the DDX54 protein or a gene encoding the same.

3. The pharmaceutical composition for the prevention or treatment of cancer of claim 1, wherein the cancer has resistance to the cancer immunotherapy agent.

4. The pharmaceutical composition for the prevention or treatment of cancer of claim 1, wherein the DDX54 inhibitor reduces resistance of cancer cells to the cancer immunotherapy agent.

5. The pharmaceutical composition for the prevention or treatment of cancer of claim 1, wherein the cancer immunotherapy agent is an immune checkpoint inhibitor, an immune cell therapy, or an immune virus therapy.

6. The pharmaceutical composition for the prevention or treatment of cancer of claim 5, wherein the immune checkpoint inhibitor is at least one selected from the group consisting of a PD-1 inhibitor, a PDL-1 inhibitor, a PD-L2 inhibitor, a CTLA-4 inhibitor, a LAG-3 inhibitor, a TIM-3 inhibitor, a CEACAM1 inhibitor, a CD27 inhibitor, a CD28 inhibitor, a CD70 inhibitor, a CD80 inhibitor, a CD86 inhibitor, a CD137 inhibitor, a CD276 inhibitor, a KIRs inhibitor, a LAG3 inhibitor, a TNFRSF4 inhibitor, a GITR inhibitor, a GITRL inhibitor, a 4-1BBL inhibitor, a A2AR inhibitor, a VTCN1 inhibitor, a BTLA inhibitor, a IDO inhibitor, a TIM-3 inhibitor, a VISTA inhibitor, a KLRA inhibitor, and combinations thereof.

7. The pharmaceutical composition for the prevention or treatment of cancer of claim 1, wherein the composition inhibits the activity of at least one signal transduction pathway selected from the group consisting of JAK-STAT3, MYC, STEMNESS, EMT, and WNT.

8. The pharmaceutical composition for the prevention or treatment of cancer of claim 1, wherein the composition increases tumor-infiltrating lymphocytes (TILs).

9. The pharmaceutical composition for the prevention or treatment of cancer of claim 1, wherein the cancer is solid cancer.

10. The pharmaceutical composition for the prevention or treatment of cancer of claim 1, wherein the cancer is at least one selected from the group consisting of breast cancer, head and neck cancer, uterine cancer, brain cancer, skin cancer, kidney cancer, lung cancer, colorectal cancer, prostate cancer, liver cancer, bladder cancer, kidney cancer, pancreatic cancer, thyroid cancer, esophageal cancer, eye cancer, stomach cancer, ovarian cancer, cervical cancer, gallbladder cancer, bile duct cancer, and osteosarcoma.

11. A composition for enhancing the efficacy of cancer immunotherapy comprising a DEAD-box Helicase 54 (DDX54) inhibitor

12. The composition for enhancing the efficacy of cancer immunotherapy of claim 11, wherein the composition reduces the resistance of cancer to the cancer immunotherapy agent and enhances anti-tumor immune responses.

13. The composition for enhancing the efficacy of cancer immunotherapy of claim 11, wherein the cancer immunotherapy agent is an immune checkpoint inhibitor.

14. The composition for enhancing the efficacy of cancer immunotherapy of claim 13, wherein the immune checkpoint inhibitor is at least one selected from the group consisting of a PD-1 inhibitor, a PDL-1 inhibitor, a PD-L2 inhibitor, a CTLA-4 inhibitor, a LAG-3 inhibitor, a TIM-3 inhibitor, a CEACAM1 inhibitor, a CD27 inhibitor, a CD28 inhibitor, a CD70 inhibitor, a CD80 inhibitor, a CD86 inhibitor, a CD137 inhibitor, a CD276 inhibitor, a KIRs inhibitor, a LAG3 inhibitor, a TNFRSF4 inhibitor, a GITR inhibitor, a GITRL inhibitor, a 4-1BBL inhibitor, a A2AR inhibitor, a VTCN1 inhibitor, a BTLA inhibitor, a IDO inhibitor, a TIM-3 inhibitor, a VISTA inhibitor, a KLRA inhibitor, and combinations thereof.

15. A method for enhancing the efficacy of cancer immunotherapy, comprising co-administering a DEAD-box Helicase 54 (DDX54) inhibitor and a cancer immunotherapy agent to a subject.

16. The method for enhancing the efficacy of cancer immunotherapy of claim 15, wherein the inhibitor is co-administered simultaneously, separately, or sequentially with the cancer immunotherapy agent.

17. A method for treating cancer comprising administering a DEAD-box Helicase 54 (DDX54) inhibitor and a cancer immunotherapy agent to a subject.
